# EUROPÄISCHE PATENTSCHRIFT

(11) **EP 3 422 975 B1**
(45) Veröffentlichungstag und Bekanntmachung des Hinweises auf die Patenterteilung: **14.10.2020**
(21) Anmeldenummer: 17713164.6
(22) Anmeldetag: 01.03.2017
(51) Int. Cl.: A61B 17/88

(54) **POSITIONIERVORRICHTUNG ZUM FIXIEREN EINER POLYAXIALEN PLATTE AN EINEM RÖHRENKNOCHEN**
POSITIONING DEVICE FOR SECURING A POLYAXIAL PLATE IN A TUBULAR BONE
DISPOSITIF DE POSITIONNEMENT POUR FIXER UNE PLAQUE POLYAXIALE SUR UN OS LONG

(30) Priorität: 01.03.2016 DE 102016103681; 02.03.2016 DE 102016103754
(43) Veröffentlichungstag der Anmeldung: 09.01.2019
(73) Patentinhaber: OT Medizintechnik GmbH, 80639 München (DE)
(72) Erfinder: SCHREIBER, Ulrich, 80639 München (DE); ZAPF, Lukas, 81927 München (DE); GATTINGER, Johannes, 84028 Landshut (DE)
(74) Vertreter: Bobbert & Partner Patentanwälte PartmbB
(86) Internationale Anmeldenummer: PCT/EP2017/054798
(87) Internationale Veröffentlichungsnummer: WO 2017/149026

(56) Entgegenhaltungen:
- EP-A2- 2 745 786
- DE-A1-102014 109 935
- ES-T3- 2 523 021
- US-A1- 2008 188 852

## Beschreibung

Die Erfindung betrifft eine Positioniervorrichtung für die Plattenosteosynthese gemäß dem Oberbegriff des Anspruchs 1.

Platten sind bekannte Hilfsmittel zur Versorgung von Brüchen beispielsweise langer Röhrenknochen, aber auch anderer Knochen. Sie werden mittels Schrauben mit dem Knochen verbunden.

Bei bisher bekannten Platten werden die Schrauben durch diskrete Öffnungen der Platte in einer vorbestimmten Stellung zur Platte in den Knochen eingeschraubt. Das exakte Platzieren der Schrauben in den Knochen setzt eine große Erfahrung des Operateurs voraus.

Aus der Offenlegungsschrift DE 10 2014 109 935 A1 ist eine Positioniervorrichtung zum Fixieren eines Marknagels in einem Röhrenknochen bekannt.

Aus der Offenlegungsschrift EP 2 745 786 A2 ist ein Gegenstand zum Bestimmen der Bewegungsbahn eines zweiten Objekts in Bezug auf ein erstes Objekt bekannt.

Aus der Offenlegungsschrift US 2008/188852 A1 ist eine Vorrichtung zum Repositionieren von Teilen eines Knochenbruchs bekannt.

Aus der Offenlegungsschrift ES 2 523 021 T3 ist ein knochenstabilisierendes Implantat mit durchgehenden Öffnungen zum Verankern des Implantats bekannt.

Aufgabe der vorliegenden Erfindung ist es, eine Positioniervorrichtung zum Fixieren einer Platte an einem Knochen oder des Knochens an einer Platte anzugeben.

Die erfindungsgemäße Aufgabe wird mit einer Positioniervorrichtung mit den Merkmalen des Anspruchs 1 gelöst.

Die erfindungsgemäße Positioniervorrichtung dient dem Positionieren und/oder Fixieren einer Platte an einem Knochen. Sie umfasst einen Führungsbogen. Der Führungsbogen ist in wenigstens einem Abschnitt als ein Steckabschnitt zum Einstecken in eine Hülse einer Steckhülsenanordnung ausgestaltet, weist einen solchen Steckabschnitt auf oder ist hiermit verbunden.

Der Führungsbogen weist auf oder ist verbunden mit eine(r) Verstellvorrichtung, wobei die Verstellvorrichtung wenigstens eine Zielvorrichtung aufweist. Die Zielvorrichtung ist ausgebildet, um eine Verriegelungsvorrichtung oder ein Instrument zum Einwirken auf die Verriegelungsvorrichtung zumindest abschnittsweise aufzunehmen.

Die Steckhülsenanordnung oder Einsteckhülsenanordnung weist wenigstens eine oder eine Vielzahl von Hülsen zum Einstecken des Steckabschnitts einer Positioniervorrichtung, insbesondere der erfindungsgemäßen Positioniervorrichtung, auf.

Bei allen vorstehenden und folgenden Ausführungen ist der Gebrauch des Ausdrucks "kann sein" bzw. "kann haben" usw. synonym zu "ist vorzugsweise" bzw. "hat vorzugsweise" usw. zu verstehen und soll erfindungsgemäße Ausführungsformen erläutern.

Wann immer hierin Zahlenworte genannt werden, so versteht der Fachmann diese als Angabe einer zahlenmäßig unteren Grenze. Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

In einigen erfindungsgemäßen, beispielhaften Ausführungsformen ist der Steckabschnitt ein Steckstab oder weist einen solchen auf.

Erfindungsgemäß weist die Positioniervorrichtung eine Steckhülsenanordnung auf, welche eine Vielzahl von Hülsen zum Einstecken des Steckabschnitts aufweist.

In einigen erfindungsgemäßen, beispielhaften Ausführungsformen weisen wenigstes zwei der Hülsen jeweils eine Längsachse auf. Nicht jede dieser Längsachsen stehen parallel zueinander.

Die Positioniervorrichtung kann in einigen erfindungsgemäßen, beispielhaften Ausführungsformen als monoaxial bezeichnet werden, wenn die Positioniervorrichtung ausgestaltet oder eingestellt ist, die Verriegelungseinrichtung, z. B. eine Knochenschraube, unter nur einem Winkel in die konkrete Durchgangsöffnung der Platte einzubringen.

In einigen erfindungsgemäßen, beispielhaften Ausführungsformen weist die Steckhülsenanordnung einen mit ihr verbundenen Plattenhalter auf. Der Plattenhalter erstreckt sich vorzugsweise von der Steckhülsenanordnung.

In einigen erfindungsgemäßen, beispielhaften Ausführungsformen weist der Plattenhalter eine erste Verbindung auf, mittels welcher der Plattenhalter lösbar mit einem weiteren Abschnitt der Steckhülsenanordnung verbunden oder verbindbar ist.

In einigen erfindungsgemäßen, beispielhaften Ausführungsformen weist der Plattenhalter eine zweite Verbindung zu seiner Verbindung mit einer Platte zur Osteosynthese auf.

In einigen erfindungsgemäßen, beispielhaften Ausführungsformen sind die erste Verbindung und/oder die zweite Verbindung als Schraubverbindungen ausgestaltet oder weisen eine Schraubenverbindung auf. Die erste Verbindung und/oder die zweite Verbindung können Kombination mehrerer Verbindungsmechanismen sein, etwa Schraubverbindungen und Steckverbindungen zugleich.

Die Platte kann polyaxial oder monoaxial oder als eine Kombination hiervon ausgeführt sein. Dasselbe kann für die erfindungsgemäße Positioniervorrichtung gelten.

Unter "polyaxial" kann verstanden werden, dass die mit der Platte in Verbindung stehende Positioniervorrichtung ausgestaltet oder eingestellt ist, die Verriegelungseinrichtung, z. B. eine Knochenschraube, unter einer Vielzahl von Winkeln, auch multidirektional genannt, in eine konkrete Durchgangsöffnung der Platte einzubringen.

Unter "monoaxial" kann verstanden werden, dass die mit der Platte in Verbindung stehende Positioniervorrichtung ausgestaltet oder eingestellt ist, die Verriegelungseinrichtung, z. B. eine Knochenschraube, unter nur einem Winkel in eine konkrete Durchgangsöffnung der Platte einzubringen.

Die Platte kann in einigen erfindungsgemäßen, beispielhaften Ausführungsformen als polyaxial bezeichnet werden, wenn die Positioniervorrichtung ausgestaltet oder eingestellt ist, die Verriegelungseinrichtung, z. B. eine Knochenschraube, unter mehr als nur einem Winkel in die konkrete Durchgangsöffnung der Platte einzubringen.

In einigen erfindungsgemäßen, beispielhaften Ausführungsformen weist der Steckabschnitt wenigstens einen sich radial hiervon erstreckenden Führungsstift auf. Der Führungsstift dient zum Führen des Steckabschnitts innerhalb einer Hülse, ausgewählt aus der Vielzahl der Hülsen. Dies kann optional entlang einer optional in den Hülsen jeweils ausgestalteten Längsnut (auch als Nut zu bezeichnen) erfolgen.

In einigen erfindungsgemäßen, beispielhaften Ausführungsformen weist jede Hülse aus der Vielzahl der Hülsen wenigstens je eine Längsnut auf.

In einigen erfindungsgemäßen, beispielhaften Ausführungsformen ist die Anordnung von Hülse und Steckabschnitt auch vertauscht. Der Steckabschnitt kann sich somit an dem als Steckhülsenanordnung oder Einsteckhülsenanordnung bezeichneten Bauteil befinden und die Hülse oder die Hülsen an der Positioniervorrichtung.

In einigen erfindungsgemäßen, beispielhaften Ausführungsformen erstreckt sich die Längsnut nicht über die gesamte Länge der Hülse. Letzteres kann eine Einführbegrenzung beim Einführen des Steckabschnitts in die Hülse sicherstellen. Ergänzend oder alternativ ist eine Breite der Längsnut nicht über die gesamte Länge der Hülse konstant. Letzteres kann ein Verklemmen des Führungsstifts in einem Endabschnitt der Nut erlauben, also z. B. eine Verklemmung, eine optimale Positionierung des Führungsbogens relativ zur Hülse und/oder zur Platte sicherstellen.

In einigen erfindungsgemäßen, beispielhaften Ausführungsformen verläuft die Längsnut wenigstens abschnittsweise auch in Umfangsrichtung der Hülse. Dies kann vorteilhaft eine Verriegelung des Steckabschnitts in der Hülse begünstigen und/oder ein ungewolltes Herausrutschen des Steckabschnitts aus der Hülse verhindern.

In einigen erfindungsgemäßen, beispielhaften Ausführungsformen weist die Längsnut wenigstens einen Passabschnitt auf, welcher mit dem Führungsstift eine vorbestimmte Passung ergibt.

In einigen erfindungsgemäßen, beispielhaften Ausführungsformen weist die Längsnut den wenigstens einen Passabschnitt in einem Endbereich der Längsnut oder in einem Bereich, in welchem ein Führungsstift nach Einstecken des Steckabschnitts in die Hülse zum Liegen kommen soll, auf. Die übrigen Abschnitte der Längsnut sind breiter als der Passabschnitt. Damit lässt sich der Führungsabschnitt leicht in die Längsnut einführen und nimmt den Passsitz erst gegen Ende des Einführens ein.

In einigen erfindungsgemäßen, beispielhaften Ausführungsformen ist die Querschnittsform der Hülse oval, rechteckig, nicht-rund, nicht-kreisförmig, eckig, dreieckig, etc. ausgestaltet.

In einigen erfindungsgemäßen, beispielhaften Ausführungsformen ist die Querschnittsform der Hülse derart ausgestaltet (oval, rechteckig, nicht-rund, nicht-kreisförmig, eckig, dreieckig, etc.), dass dadurch die Achse und die Ausrichtung des Führungsstifts und damit des Führungsbogens um diese Achse bestimmt ist.

In einigen erfindungsgemäßen, beispielhaften Ausführungsformen ist die Höhenbegrenzung des Führungsstifts und damit des Führungsbogens entlang der Achse der Hülse und somit des Führungsstifts über eine Passung an der Stirnseite der Hülse gegenüber eines Absatzes des Führungsstifts bestimmt.

In einigen erfindungsgemäßen, beispielhaften Ausführungsformen ist die Höhenbegrenzung des Führungsstifts und damit des Führungsbogens entlang der Achse der Hülse und somit des Führungsstifts über eine Passung an der Stirnseite der Hülse gegenüber eines in axialer Richtung verschieblichen oder einstellbaren Absatzes des Führungsstifts bestimmt.

In einigen erfindungsgemäßen, beispielhaften Ausführungsformen ist die Höhenbegrenzung des Führungsstifts und damit des Führungsbogens entlang der Achse der Hülse und somit des Führungsstifts über eine Passung an der Stirnseite der Hülse gegenüber einem unterschiedlich langen Abstandhalter zwischen Führungsstift und Hülse bestimmt.

In einigen erfindungsgemäßen, beispielhaften Ausführungsformen ist die Vielzahl von Hülsen mittels Markierungen auf der Steckhülsenanordnung bezeichnet. Die Bezeichnung kann angeben, auf welche Öffnung oder Durchgangsöffnung der Platte die in der Zielvorrichtung der Verstellvorrichtung aufgenommene Verriegelungsvorrichtung ausgerichtet ist. Dies kann dem Operateur die Arbeit erleichtern. Hülsen und Öffnung oder Durchgangsöffnung können eineindeutig markiert sein, so dass eine Zuordnung einer konkreten Hülse zu - vorzugsweise nur - einer konkreten Durchgangsöffnung möglich ist.

Die Markierungen auf der Platte können ausgestaltet sein, um mittels Durchleuchtung oder Röntgen ausgelesen oder erkannt zu werden.

In einigen erfindungsgemäßen, beispielhaften Ausführungsformen ist die Vielzahl von Hülsen mit anderen Abschnitten der Steckhülsenanordnung verschraubt, mittels Passung verbunden, mittels Kleber und/oder mittels Passstift verbunden.

In einigen erfindungsgemäßen, beispielhaften Ausführungsformen hat die Vielzahl von Hülsen je wenigstens eine Öffnung für wenigstens einen Passstift.

In einigen erfindungsgemäßen, beispielhaften Ausführungsformen weist der Steckabschnitt in seiner Mantelfläche eine Durchgangsöffnung zum Durchführen eines Führungsstifts auf. Der Steckabschnitt weist in einem Inneren hiervon wenigstens eine Längsöffnung auf, wobei im Inneren oder in der Längsöffnung ein Stab drehbar angeordnet ist, welcher auf seiner Mantelfläche den Führungsstift trägt oder von welcher der Führungsstift absteht oder wegsteht, wobei der Führungsstift durch die Durchgangsöffnung geführt ist.

In einigen erfindungsgemäßen, beispielhaften Ausführungsformen weist die Hülse, z. B. in ihrer Mantelfläche, wenigstens oder genau einen Führungs- oder Passstift als

Verdrehsicherung auf. Der Steckabschnitt kann wenigstens eine Nut oder Längsöffnung aufweisen.

In einigen erfindungsgemäßen, beispielhaften Ausführungsformen weist der Stab ein Handelement zum Drehen des Stabs von Hand innerhalb der Längsöffnung des Steckabschnitts auf.

In einigen erfindungsgemäßen, beispielhaften Ausführungsformen ist die Positioniervorrichtung verbunden mit der Steckhülsenanordnung. Die Steckhülsenanordnung ist ihrerseits verbunden mit einer Platte für die Osteosynthese. Die Platte weist eine Vielzahl von Öffnungen oder Durchgangsöffnungen zur Aufnahme einer oder mehrerer Verriegelungsvorrichtungen auf. Die Tiefe der Durchgangsöffnungen entspricht jeweils einer Dicke der Platte an der Stelle der Durchgangsöffnung. Aus der Vielzahl der Hülsen ist jeweils wenigstens eine einer oder mehreren der Durchgangsöffnungen zugeordnet. Die Verstellvorrichtung ist derart ausgestaltet, dass bei in eine der Hülsen eingeführtem Steckabschnitt - mit vorzugsweise, falls vorgesehen, verriegeltem Führungsstift - die in der Verstellvorrichtung aufgenommene Zielvorrichtung auf vorbestimmte Weise ausgerichtet und bewegbar ist. Diese vorbestimmte Bewegung ist optional derart festgelegt, dass eine in der Zielvorrichtung aufgenommene Verriegelungsvorrichtung oder ein in der Zielvorrichtung aufgenommenes Instrument innerhalb eines Kegelmantels bewegbar ist. Die Kegelspitze kommt dabei innerhalb der Tiefe jener Durchgangsöffnung zum Liegen, auf welche die Verriegelungsvorrichtung durch Auswahl der zugehörigen Hülse, in welche der Steckabschnitt eingeführt ist, angesetzt ist.

Vorzugsweise kommt die Spitze des virtuellen oder rechnerisch ermittelbaren Kegels auf einer Längsachse der entsprechenden Durchgangsöffnung zum Liegen, also z. B. in der Lochmitte bei Draufsicht auf die Durchgangsöffnung.

Wann immer hier von Kegel die Rede ist, soll dies nicht beschränkend sein. Als "Kegelspitze" kann auch der Schnitt von zwei oder mehr Einbringungsrichtungen zu verstehen sein, die sich durch Bewegen der Verriegelungseinrichtung mittels der Verstelleinrichtung erzielen lassen. Auf eine tatsächliche Kegelform kommt es hier nicht zwingend an.

In einigen erfindungsgemäßen, beispielhaften Ausführungsformen weist die Steckhülsenanordnung eines oder mehrere der hierin im Zusammenhang mit der oder den Hülsen beschriebenen Merkmale in beliebiger Kombination auf, sofern eine solche Kombination für den Fachmann nicht erkennbar technisch unmöglich ist.

Die Steckhülsenanordnung und/oder Einsteckhülsenanordnung kann zum minimalinvasiven Einbringen der Platte an einem Knochen verwendet werden.

Der Führungsbogen kann als minimalinvasive Einbringhilfe der Platte an dem Knochen verwendet und in seiner Ausrichtung zur Platte variabel eingestellt werden.

Der Führungsbogen kann zum Positionieren und/oder Fixieren von Drähten, auch als K-Drähte oder Kirschnerdrähte bezeichnet, durch oder von einer Platte an einem Knochen verwendet werden.

Die erfindungsgemäße Positioniervorrichtung mit der Verbindung zwischen Platte und Steckhülsenanordnung, der Steckhülsenanordnung und dem Führungsbogen mit Verstelleinrichtung können aus röntgendurchlässigen Materialien gefertigt sein.

Manche oder alle erfindungsgemäßen Ausführungsformen können einen, mehrere oder alle der oben und/oder im Folgenden genannten Vorteile aufweisen.

Mittels der erfindungsgemäßen Positioniervorrichtung können vorteilhaft geringfügige Fehlausrichtungen beim Positionieren und/oder beim Einschrauben (dem sogenannten "Setzen") von Verriegelungsschrauben in eine Platte während einer Operation korrigiert werden.

Mittels der erfindungsgemäßen Positioniervorrichtung können vorteilhaft Fehlausrichtungen des Knochens durch das Setzen einer Verrieglungsschraube und ein anschließendes Repositionieren (der sogenannten "Umstellungsosteotomie") durch die Platte während einer Operation korrigiert werden.

Die Lage und der Winkel von Bohrungen für die Verriegelungsschrauben und deren Lage können mittels der erfindungsgemäßen Positioniervorrichtung vorteilhaft noch intraoperativ an die individuelle anatomische und sich aus einer Verletzung ergebenden Situation angepasst werden.

Weiterhin kann vorteilhaft mittels der erfindungsgemäßen Positioniervorrichtung der Winkel der die Platte durchdringenden Verriegelungsschrauben noch intraoperativ variiert werden, um beispielsweise Frakturfragmente zu repositionieren, anatomisch korrekt zu adaptieren, oder im Rahmen einer Umstellungsosteotomie die Knochen zueinander neu zu positionieren.

Erfindungsgemäß kann die Positioniervorrichtung mit der zu fixierenden Platte sicher und einfach lösbar verbunden werden. Somit kann die Positioniervorrichtung von der fixierten Platte entkoppelt und entfernt werden. Die Steckhülsenanordnung kann vorteilhaft sicherstellen, dass sich die Positioniervorrichtung während des Verschraubens oder während des Lösens der Verschraubung zwischen Knochen und Platte, vorteilhaft nicht relativ zur Platte dreht.

Erfindungsgemäß kann die Positioniervorrichtung die Möglichkeit einer minimalinvasiven Implantation von polyaxialen Osteosyntheseplatten bieten. Der Operateur kann mittels der Steckhülsenanordnung die Positioniervorrichtung auf eine oder mehrere konkrete Durchgangsöffnung(en) ausrichten, selbst wenn er diese ebenso wie die Platte im Übrigen aufgrund des minimal(invasiv)en Zugangs nicht sehen kann. Der Operateur kann sich hierbei der Durchleuchtung oder des Röntgens bedienen, was aber in sein Belieben gestellt ist.

Die Positioniervorrichtung mit der Steckhülsenanordnung lässt sich intuitiv handhaben. Zum Umsetzen der Positioniervorrichtung innerhalb der Steckhülsenanordnung bedarf es vorteilhaft nur eines Schrittes, nämlich des Umsteckens des Steckabschnitts von einer Hülse in eine andere.

Die vorliegende Erfindung wird im Folgenden anhand der beigefügten Figuren, in welcher identische Bezugszeichen gleiche oder ähnliche Bauteile bezeichnen, exemplarisch erläutert. In den jeweils schematisch vereinfachten Figuren gilt:
- **Fig. 1**: zeigt eine erfindungsgemäße Positioniervorrichtung einer ersten beispielhaften Ausführungsform in einer perspektivischen Ansicht;
- **Fig. 2**: zeigt die erfindungsgemäße Positioniervorrichtung aus Fig. 1 in einer anderen perspektivischen Ansicht;
- **Fig. 3**: zeigt die Steckhülsenanordnung einer ersten beispielhaften Ausführungsform in einer perspektivischen Ansicht;
- **Fig. 4**: zeigt die Steckhülsenanordnung der Fig. 3 von oben;
- **Fig. 5**: zeigt Teile der erfindungsgemäßen Positioniervorrichtung aus Fig. 1 sowie vier Hülsen;
- **Fig. 6**: zeigt die erfindungsgemäße Positioniervorrichtung aus Fig. 1 verbunden mit einer Platte in einer perspektivischen Ansicht;
- **Fig. 7**: zeigt die erfindungsgemäße Positioniervorrichtung aus Fig. 6 im Wesentlichen von der Seite;
- **Fig. 8**: zeigt einen Steckabschnitt für eine erfindungsgemäße Positioniervorrichtung einer zweiten Ausführungsform;
- **Fig. 9**: zeigt schematisch die Ausrichtung zwischen Positioniervorrichtung und Platte in einer exemplarischen, erfindungsgemäßen Ausführungsform;
- **Fig. 10**: zeigt den Plattenhalter aus Fig. 6 oder 7 verbunden mit einer monoaxialen Positioniervorrichtung in einer perspektivischen Ansicht;
- **Fig. 11**: zeigt den Plattenhalter der Fig. 10 verbunden mit einer monoaxialen Positioniervorrichtung in einer Seitenansicht;
- **Fig. 12**: zeigt eine weitere Ausführungsform einer erfindungsgemäßen Positioniervorrichtung;
- **Fig. 13**: zeigt einen Drehspanner für die erfindungsgemäße Positioniervorrichtung;
- **Fig. 14a**: zeigt einen Steckabschnitt für eine erfindungsgemäße Positioniervorrichtung einer dritten Ausführungsform;
- **Fig. 14b**: zeigt einen Steckabschnitt für eine erfindungsgemäße Positioniervorrichtung einer weiteren Ausführungsform mit eckigen Hülsen;
- **Fig. 14c**: zeigt einen Steckabschnitt für eine erfindungsgemäße Positioniervorrichtung einer weiteren Ausführungsform mit Zwischenhülsen;
- **Fig. 14d**: zeigt einen Steckabschnitt für eine erfindungsgemäße Positioniervorrichtung einer weiteren Ausführungsform mit einer arretierbaren Zwischenhülse;
- **Fig. 14e**: zeigt einen Steckabschnitt für eine erfindungsgemäße Positioniervorrichtung einer weiteren Ausführungsform mit einer Arretiervorrichtung;
- **Fig. 15**: zeigt die erfindungsgemäße Positioniervorrichtung zum Führen von K-Drähten in einer Seitenansicht; und
- **Fig. 16**: zeigt die erfindungsgemäße Positioniervorrichtung in einer weiteren Ausführungsform.

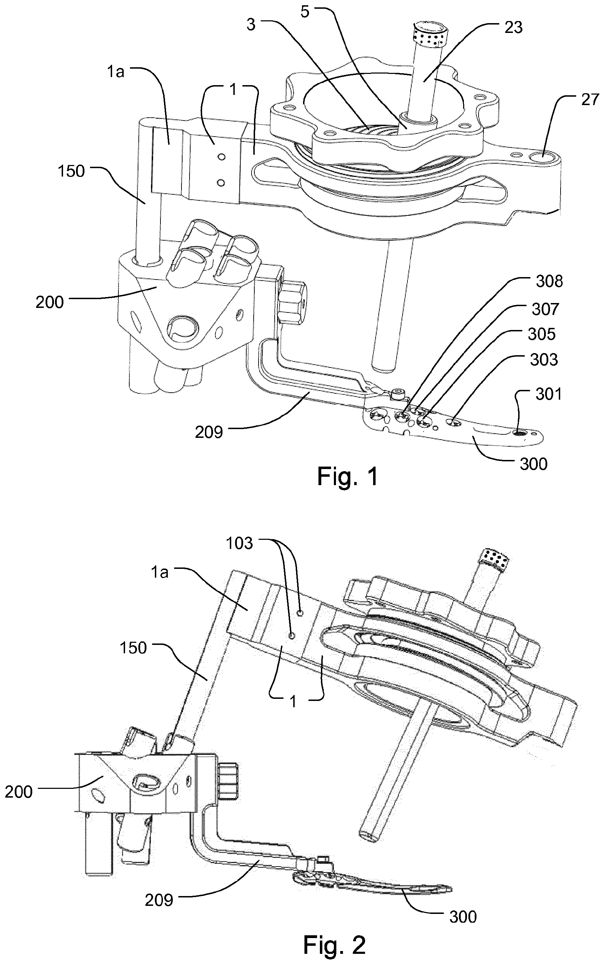

**Fig. 1** zeigt eine erfindungsgemäße Positioniervorrichtung 100 einer ersten beispielhaften Ausführungsform in einer perspektivischen Ansicht.

Die Positioniervorrichtung 100 kann dabei auf jede Weise ausgestaltet sein, die in der WO 2016/008849 A1 offenbart ist.

Fig. 1 zeigt ferner eine Steckhülsenanordnung 200, welche einerseits mit der Positioniervorrichtung 100, nämlich durch Einstecken eines Steckabschnitts 150 der Positioniervorrichtung 100 in eine der Hülsen 201, 203, 205 und 207 der Steckanordnung 200, und andererseits mit einer Platte 300 lösbar verbunden ist.

Die Platte 300 weist Durchgangsöffnungen 301, 303, 305, 307, 308 zum Verschrauben oder Verriegeln der Platte 300 mit einem Knochen auf.

Eine Längsachse oder Rotationsachse der Durchgangsöffnungen 301, 303, 305, 307, 308 steht dabei vorzugsweise parallel zu einer Längsachse wenigstens oder genau einer der Hülsen 201, 203, 205 und 207, und ggf. weiterer, der Steckhülsenanordnung 200.

Das Instrument 23 zum Einführen von Schrauben kann wahlweise und je nach gewünschter Fixierung und Verschraubung der Platte 300 am Knochen in eine Positionierhilfe 27 in dem Führungsbogen 1 eingeführt werden.

**Fig. 2** zeigt die erfindungsgemäße Positioniervorrichtung 100 aus Fig. 1 in einer weiteren perspektivischen Ansicht.

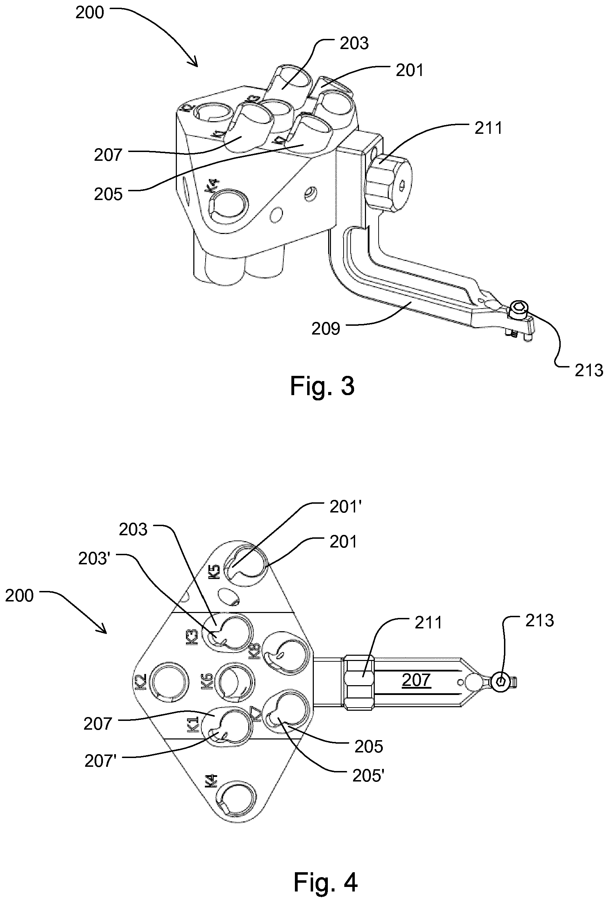

**Fig. 3** zeigt die Steckhülsenanordnung 200 einer ersten beispielhaften Ausführungsform in einer perspektivischen Ansicht.

**Fig. 4** zeigt die Steckhülsenanordnung 200 der Fig. 3 von oben.

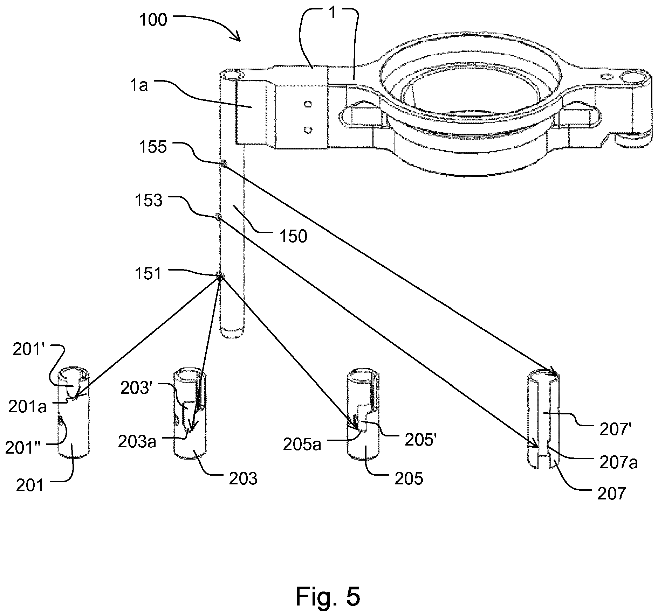

**Fig. 5** zeigt Teile der erfindungsgemäßen Positioniervorrichtung 100 aus Fig. 1 sowie vier Hülsen 201, 203, 205 und 207.

Fig. 5 zeigt verschiedene Lösungen zum Verbinden der Positioniervorrichtung 100 mit den Hülsen der in Fig. 5 nicht gezeigten Steckhülsenanordnung. Gezeigt sind in Fig. 5 allein Hülsen 201, 203, 205 und 207, welche Teil der in Fig. 3 gezeigten Steckhülsenanordnung 200 sein können.

Die Hülsen 201, 203, 205 und 207 weisen jeweils eine Nut 201', 203', 205' und 207' auf, welche zum Aufnehmen der Führungsstifte 151, 153 und/oder 155 des Steckabschnitts 150 dienen können.

Die Nuten 201', 203', 205' der Hülsen 201, 203, 205 sind ausgestaltet, um den Steckabschnitt 150 mittels nur eines Führungsstifts 151 sowohl in dessen Höhe als auch Rotation (jeweils bezogen auf die Hülse) festzulegen.

Hierzu können die Nuten 201', 203', 205' jeweils einen optionalen, kurvigen Verlauf aufweisen und/oder einen endseitigen Passabschnitt 201a, 203a oder 205a, in welchen der Führungsstift 151 passend eingeführt werden kann. Passabschnitte 201a, 203a oder 205a einerseits und Führungsstift 151 andererseits können optional gemeinsam ein Zentrieren bewirken.

Am Beispiel der Hülse 201 ist gezeigt, dass jede Hülse wenigstens eine Öffnung 201' zur Aufnahme wenigstens eines Passstifts aufweisen kann, mittels welchem die Hülse 201 an der Steckhülsenanordnung 200 befestigt und/oder ausgerichtet sein kann.

Am Beispiel der Hülse 207 ist hingegen gezeigt, dass Höhe und Rotationswinkel des Steckabschnitts 150 auch mittels mehr als nur eines Führungsstifts sichergestellt werden können. Die Nut 207', welche einen Passabschnitt 207a aufweist, kann mittels eines Führungsstifts 153 für einen Schutz vor ungewollter Rotation dienen. Ein zweiter Führungsstifts 155 kann, beispielsweise durch Aufliegen auf dem oberen Rand der Hülse 207 ein zu tiefes Einstecken des Steckabschnitts 150 in die Hülse 207 vermeiden. Alternativ kann der zweite Führungsstift 155 auf andere Weise mit der Hülse 207 in Kontakt stehen oder mit dieser verbunden sein.

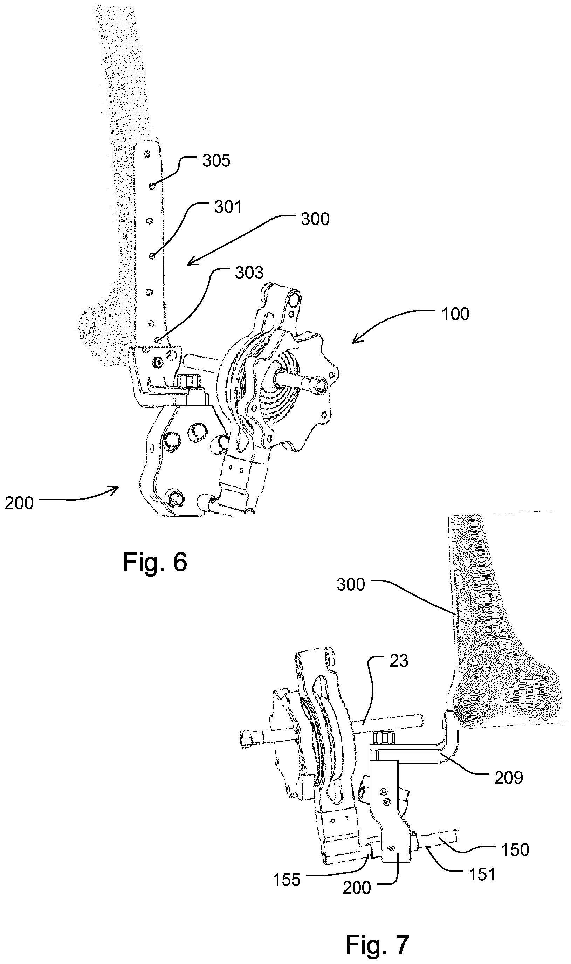

Wie nicht zuletzt in Fig. 7 zu erkennen ist, kann die nach unten offene Nut 207a, deren Breite größer als die Dicke des Führungsstifts 151 ist, ein Durchschieben des Führungsstifts 151 durch den Passabschnitt 207a erlauben. Erst der Führungsstift 155 kann den Passabschnitt 207a nicht überwinden. Auf diese Weise kann der Steckabschnitt 150 vergleichsweise sehr tief in die Hülse 207 eingeschoben werden, was bei anderen Hülsen, etwa bei der Hülse 201, nicht der Fall ist. Diese Ausgestaltung erlaubt eine große Flexibilität hinsichtlich des Abstands der Positioniervorrichtung 100 von der Platte 300.

**Fig. 6** zeigt die erfindungsgemäße Positioniervorrichtung aus Fig. 1, verbunden mit einer Platte 300 in einer perspektivischen Ansicht;

**Fig. 7** zeigt die erfindungsgemäße Positioniervorrichtung aus Fig. 6 im Wesentlichen von der Seite.

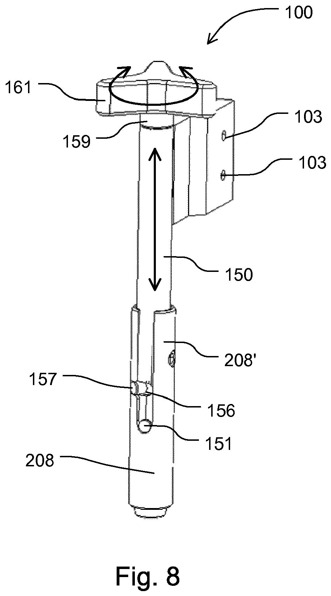

**Fig. 8** zeigt einen Steckabschnitt 150 für eine erfindungsgemäße Positioniervorrichtung 100 einer zweiten Ausführungsform.

In Fig. 8 weist der Steckabschnitt 150 neben dem aus den vorangegangenen Figuren bekannten Führungsstift 151 einen weiteren Führungsstift 157 auf. Dieser ragt aus einer Mantel- oder Umfangsfläche des Steckabschnitts 150 hervor und ist mit einem im Inneren des Steckabschnitts 150 gelegenen Stab 159 optional rotationsfest verbunden. Wird der Stab 159 mittels des optionalen Handelements 161 in Pfeilrichtung gedreht, so verriegelt der Führungsstift 157 den Steckabschnitt 150 in dem in Umfangsrichtung verlaufenden Abschnitt 208b der Nut 208' der Hülse 208. Er sichert so gegen Herausrutschen des Steckabschnitts 150 aufgrund einfachen Bewegens oder ungewollten Rotierens der Positioniervorrichtung 100.

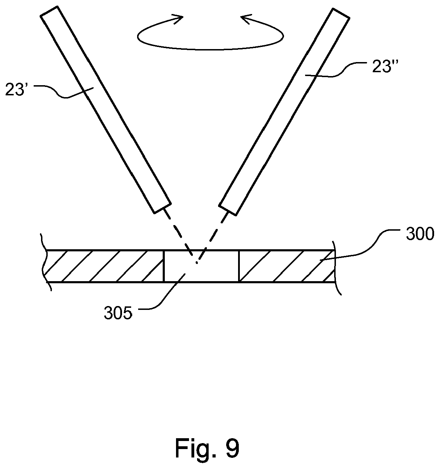

**Fig. 9** zeigt schematisch die Ausrichtung zwischen Positioniervorrichtung und Platte in einer exemplarischen, erfindungsgemäßen Ausführungsform. Die Ausrichtung ist dergestalt, dass das Instrument 23 (oder jeder andere Gegenstand, der durch die Zielvorrichtung 5, welche in Fig. 9 nicht gezeigt ist) zwischen Endlagen, von welchen zwei Lagen 23' und 23" gezeigt sind, einen Kegel umschreibend bewegt werden kann.

Die Spitze des Kegels soll dabei in der Dicke der Platte 300, welche hier im Schnitt von der Seite gezeigt ist, oder einer der Öffnungen 305 für die nicht gezeigte Verriegelungsvorrichtung zum Liegen kommen, wie mittels Strichlinien gezeigt.

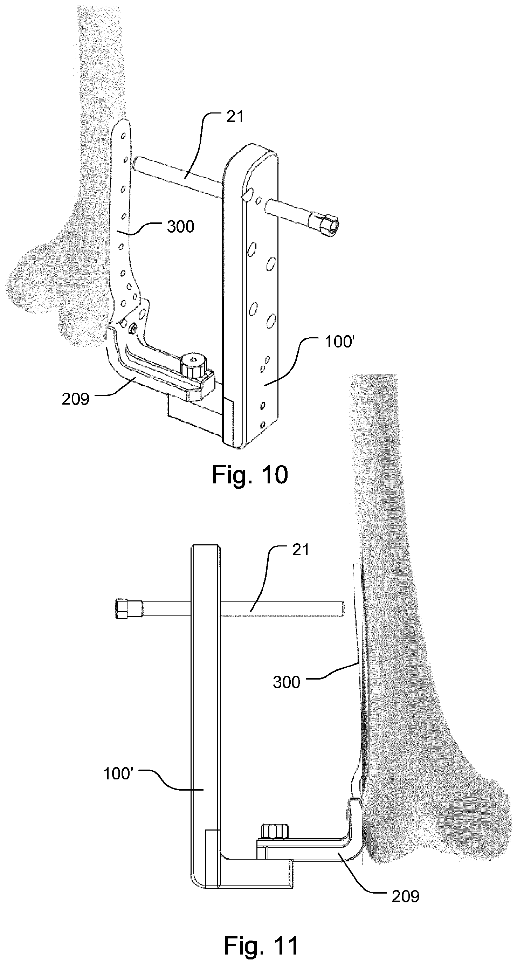

**Fig. 10** zeigt den Plattenhalter 209 aus Fig. 6 oder 7, verbunden mit einer monoaxialen Positioniervorrichtung 100' in einer perspektivischen Ansicht.

**Fig. 11** zeigt den Plattenhalter 209 der Fig. 10 in ein Seitenansicht.

Die Ausführungsform kann ein Set betreffen mit oder bestehend aus der polyaxialen Positioniervorrichtung 100 und/oder der monoaxialen Positioniervorrichtung 100', der Steckhülsenanordnung 200, dem Plattenhalter 209 und optional einer oder mehrerer Platten 300.

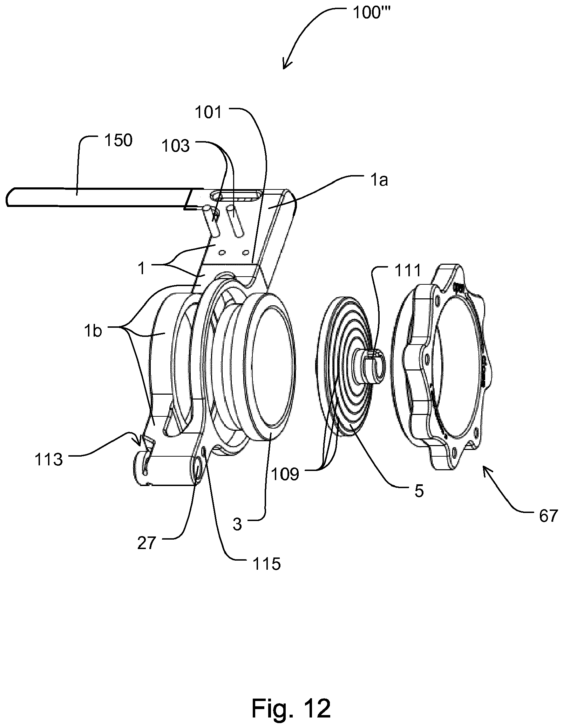

**Fig. 12** zeigt eine weitere Ausführungsform einer erfindungsgemäßen Positioniervorrichtung 100"'.

Die Verstellvorrichtung 3 ist optional als Abschnitt einer Kugeloberfläche mit einer kreisrunden Öffnung ausgeführt. Die Form der Verstellvorrichtung 3 kann anstatt einer kugelförmigen Oberfläche gleichfalls eine anders gestaltete, nur optional gekrümmte oder gerade Oberfläche aufweisen.

Die Verstellvorrichtung 3 ist optional auf dem Führungsbogen 1 fixiert.

Die beispielsweise kreisrunde Öffnung in einem zentralen oder mittleren Bereich der Verstellvorrichtung 3 ist zur Führung der Zielvorrichtung 5 und des Instruments 23 (welches zum Einsetzen der Verriegelungsschraube 21 dient) vorgesehen. Die Längsachse des Instruments 23 steht senkrecht zur Kugeloberfläche. Die Verlängerung der Längsachse des Instruments 23 verläuft durch den Mittelpunkt einer Bohrung oder Durchgangsöffnung der Platte 300.

Das Instrument 23 wird in der Zielvorrichtung 5 geführt. Die Zielvorrichtung 5 ist optional schalenförmig mit ringförmig konzentrisch angeordneten Nuten, Rillen oder Erhebungen um die mittlere Führung des Instruments 23 herum ausgeführt. Die schalenförmige Zielvorrichtung 5 kann als Mittelschale bezeichnet werden, die, in radialer Richtung betrachtet, zwischen der äußeren Schale, welche der Verstellvorrichtung 3 entspricht, und der inneren Schale, welche der schalenförmig anliegenden Oberfläche des Führungsbogens 1 entspricht, angeordnet ist.

Die Zielvorrichtung 5, also die mittlere Schale, kann zwischen der inneren und der äußeren Schale bewegt oder verschoben werden. Bei dieser Bewegung greifen optionale, in Radialrichtung federnd gelagerte, nicht gezeigter Stifte oder Pins in die ringförmigen Nuten oder Rillen der Zielvorrichtung 5 ein. Damit wird eine definierte und exakte Positionierung des Instruments 23, und damit der Verriegelungsschraube 21, möglich. Die Nutabstände zueinander entsprechen einer bestimmten und vorgegebenen Auslenkung der Zielvorrichtung 5, die in einem Winkel oder einer Gradzahl angegeben werden kann. In diesem Ausführungsbeispiel entspricht die Auslenkung zwischen zwei ringförmigen Nuten einem Winkel oder einer Gradzahl von einem Grad (1°). Dieses Prinzip oder Konzept mit federnd gelagerten Stiften, die in Nuten eingreifen, kann als Feder-Stift-Konzept bezeichnet werden.

Die Anordnung in Fig. 12 ermöglicht eine Auslenkung des Instruments 23 (und damit der Verriegelungsschraube 21) von einer gewünschten oder vorgegebenen Gradzahl (oder einem Gradbereich), beispielsweise von zehn Grad (10°) gegenüber der zentrischen Ausgangsposition in der Mitte (diese Ausgangsposition wird in den Fig. 7, 10 und 11 gezeigt und als Null-Grad-Position (0°) bezeichnet).

Mittels eines optionalen, kombinierten Formschlusses (zwischen den Stiften und den ringförmigen Nuten der Zielvorrichtung 5) und eines Reibschlusses (zwischen der mittleren und der äußeren Schale) wird ein Fixieren der Position des Instrumentes 23 zum Einschrauben oder Setzen der Verriegelungsschraube 21 ermöglicht.

Die Zielvorrichtung 5 kann optional einem kreisrunden Abschnitt einer Struktur aufliegen, welche unter der Zielvorrichtung 5 angeordnet ist. Auf diese Weise kann sichergestellt werden, dass die Zielvorrichtung 5 vorzugsweise unter gleichbleibendem Abstand zu einem Zielpunkt geführt wird.

Die Zielvorrichtung 5 wird optional mittels eines sogenannten Drehspanners 67 zwischen der Verstellvorrichtung 3 und dem zweiten Abschnitt des Führungsbogens 1 fixiert.

Die Zielvorrichtung 5 kann wie in Fig. 12 als Option gezeigt auf wenigstens einer Oberfläche Markierungen wie z. B. die exemplarisch gezeigten konzentrische Ringe 109 aufweisen. Diese optional rein optischen Ringe 109, welche alternativ oder ergänzend erhoben (oder vertieft) sein können, dienen dem Benutzer der optischen und/oder haptischen Orientierung der aktuellen Positionierung der Zielvorrichtung 5, die verschiebbar zwischen dem Drehspanner 67 und der Verstellvorrichtung 3 angeordnet ist.

Anstelle oder ergänzend zu den konzentrischen Ringen 109 kann eine mechanische Hilfe für den Arzt vorgesehen sein, beispielsweise eine Einrasteinrichtung, die bei Einrasten eine in der Hand spürbare Rückmeldung für eine vorbestimmte Winkelstellung, z. B. 0°, gibt. Eine solche, optische oder haptische Orientierung kann hilfreich sein bei Verwenden von monoaxialen Platten oder bei der monoaxialen Implantation.

Die mechanische Hilfe kann eine Nut sein, in welche ein Stift einfährt, oder dergleichen.

Der Führungsbogen 1 ist optional zweigeteilt ausgeführt. Ein erster Abschnitt 1a (in Fig. 12 oben) ist mit einem zweiten Abschnitt 1b, insbesondere wieder lösbar, verbunden. Alternativ kann die Verbindung eine nicht wieder lösbare stoffschlüssige Verbindung sein, z. B. eine Lötverbindung, eine Schweißverbindung oder eine Klebverbindung. Die stoffschlüssige, integrale Verbindung kann ein einstückiges Bauteil sein, z. B. aus einem Material mittels Guß und/oder spanender Bearbeitung hergestellt. Der erste Bereich 1a kann mittels eines generativen Herstellungsverfahrens, z. B. mittels eines Lasersinterverfahrens oder eines Rapid Prototyping Verfahrens hergestellt sein.

Ein zweiter Abschnitt 1b des Führungsbogens 1 kann einteilig oder mehrteilig hergestellt sein.

Der erste Abschnitt 1a und der zweite Abschnitt 1b können mittels einer formschlüssigen und/oder einer stoffschlüssigen Verbindung an einer Schnittstelle 101 miteinander verbunden sein. Beispielsweise kann der zweite Abschnitt 1b mittels eines Absatzes in einen hohlen Endabschnitt des ersten Abschnitts 1a formschlüssig hineingeschoben werden. Anschließend kann diese formschlüssige Verbindung mittels eines oder mehrerer Bolzen 103 (die Bolzen 103 können Passstifte sein) fixiert und gesichert werden. Diese formschlüssige Verbindung kann zusätzlich mittels einer Klebung gesichert werden. Eine Klebung kann vorteilhaft sein, um eine spielfreie Verbindung auch nach längerem Gebrauch und mehrfachen mechanischen Belastungen zu sichern. Eine spielfreie Verbindung kann für eine exakte Positionierung und/oder Fixierung von Platten 300 mittels der erfindungsgemäßen Positioniervorrichtung 100"' für einen Therapieerfolg wichtig sein.

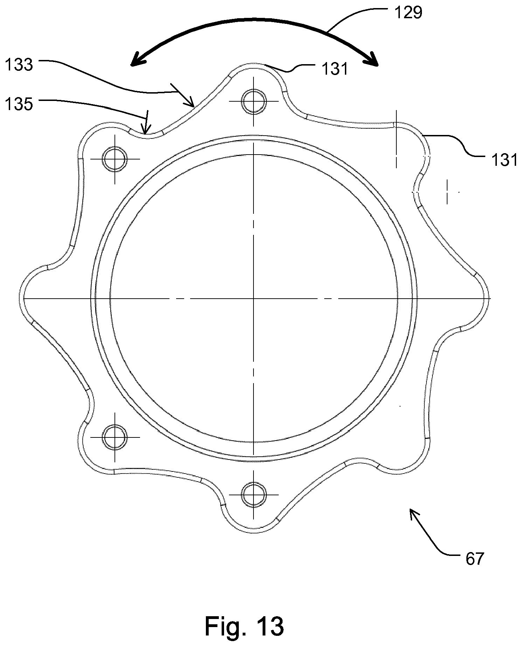

Der Drehspanner 67 verbindet und fixiert im montierten Zustand optional die Zielvorrichtung 5 und die Verstellvorrichtung 3 mit dem Führungsbogen 1. Der exemplarische Drehspanner 67 wird zu Fig. 13 näher erläutert.

Die Zielvorrichtung 5 weist auf wenigstens einer Oberfläche (in Fig. 12 auf der rechten Seite) die konzentrischen Ringe 109 auf. Diese optional rein optischen und/oder haptischen Ringe dienen dem Benutzer der Orientierung der aktuellen Positionierung der Zielvorrichtung 5, die verschiebbar zwischen dem Drehspanner 67 und der Verstellvorrichtung 3 angeordnet ist. Weiterhin weist die Zielvorrichtung 5 auf dem Umfang des zentrischen, hülsenförmigen Ansatzes optional einen Längsschlitz 111 auf. Dieser Längsschlitz 111 dient der elastischen Verformung des ringförmigen Ansatzes bei einem Einführen eines Instruments zum Einsetzen der Verriegelungsschraube 23 (siehe Fig. 1). Im nicht verformten Zustand ist der Innendurchmesser des Ansatzes geringfügig kleiner als der Durchmesser des Instruments 23. Der Ansatz wird bei dem Einführen des Instruments 23 elastisch verformt und aufgeweitet, und kann anschließend aktiv, mittels Kraftaufwand, gegen den Reibwiderstand zwischen Ansatz und Instrument verschoben oder gedreht werden. Aufgrund des Reibwiderstands kann das Instrument nur aktiv bewegt werden, nicht jedoch herausfallen. Diese Art der Klemmung ist vorteilhaft, wenn das Instrument 23 nicht ständig manuell fixiert und gehalten werden kann, aber trotzdem in einer vorgegebenen Position verbleiben soll.

Der zweite Abschnitt 1b des Führungsbogens 1 weist eine optionale Positionierhilfe 27 als Bohrung für weitere Instrumente (nicht gezeigt) zum Einsetzen von Verriegelungsschrauben auf. Diese Positionierhilfe 27 weist an einem Ende (in Fig. 12 links) ebenfalls einen Längsschlitz 113 (verdeckt) auf, der dieselbe Funktion wie der Längsschlitz 111 hat. Somit ist ein Instrument 23, welches in die Bohrung der Positionierhilfe 27 hineingeschoben wird, einerseits bewegbar, wird jedoch andererseits aufgrund des Reibwiderstands geklemmt, um ein Herausfallen zu verhindern.

Weiterhin weist der zweite Abschnitt 1b eine optionale Bohrung 115 für einen Verbindungsstift, insbesondere für einen Passstift, auf. Mittels des Passstifts kann der zweite Abschnitt 1b insbesondere mit einer Verlängerung (in Fig. 12 nicht dargestellt) verbunden werden, um beispielsweise weitere Bohrungen für Positionierhilfen zum Einsetzen von weiteren Verriegelungsschrauben in die Platte 300 vorzusehen.

Die in Fig. 12 dargestellten Bauteile können aus einem oder verschiedenen Materialien hergestellt sein. Vorzugsweise sind die Bauteile zweiter Abschnitt 1b, Verstellvorrichtung 3, Zielvorrichtung 5 und Drehspanner 67 aus einem Kunststoff hergestellt, die übrigen Bauteile aus einem oder verschiedenen metallischen Werkstoffen. Rein exemplarisch können die Bauteile aus Kunststoff aus einem oder verschiedenen der folgenden Kunststoffe hergestellt sein oder diese aufweisen: PEEK (Polyetheretherketon); PEEK faserverstärkt; PEEK faserverstärkt in unterschiedlichen Konzentrationen der Fasern; Polyoxymethylen (POM); kohlenstofffaserverstärkter Kunststoff (CFK); Polyarylsulfon, insbesondere Polyphenylsulfon (PPSU). Rein exemplarisch sind die Bauteile aus Metall aus einem Edelstahl hergestellt oder weisen dieses auf. Der Edelstahl kann gehärtet und/oder gestrahlt sein.

**Fig. 13** zeigt einen Drehspanner 67. Der Drehspanner 67 weist in dieser Ausführungsform unsymmetrische Eingriffskonturen oder Umfangs- oder Außenkonturen zum, insbesondere manuellen, Festziehen und Lösen auf.

Die optionale, unsymmetrische Eingriffskontur der Fig. 13 kann als Sägezahnkontur bezeichnet werden. Der Drehspanner 67 wird beispielsweise im Uhrzeigersinn (bezogen auf die Aufsicht in Fig. 13) der Drehrichtung 129 festgezogen und entgegen dem Uhrzeigersinn gelöst. Die Flanke im Uhrzeigersinn ist für diese Bedienung deutlich flacher als die Flanke zum Lösen. Damit kann zum Festziehen nur ein geringes Drehmoment aufgebracht werden. Wird das Drehmoment zu stark erhöht, rutschen die Hand oder die Finger im Eingriff bei einem manuellen Festziehen durch den Nutzer über die Noppen 131 hinaus. Damit kann vorteilhaft erreicht werden, dass keine zu hohen Drehmomente zum Festziehen des Drehspanners aufgebracht werden können. Sehr hohe Drehmomente könnten eine Beschädigung oder ein Bruch dieses, vorzugsweise in Kunststoff, hergestellten Bauteils verursachen. Vorzugsweise ist es bei der vorliegenden Ausgestaltung einfacher, Drehmoment zum Lösen als Drehmoment zum Festziehen auf den Drehspanner aufzubringen. Der Nutzer kann sich daher sicher sein, den von ihm manuell festgezogenen Drehspanner mit eigener Kraft auch wieder per Hand lösen zu können.

Alternativ zu einer rein manuellen Betätigung des Drehspanners 67 kann weiterhin ein Werkzeug benutzt werden.

Die unterschiedlichen Steigungen der Flanken werden mittels der Radien 133 und 135 definiert. Rein exemplarisch kann der Radius 133 ca. 6 mm und der Radius 135 ca. 49 mm betragen.

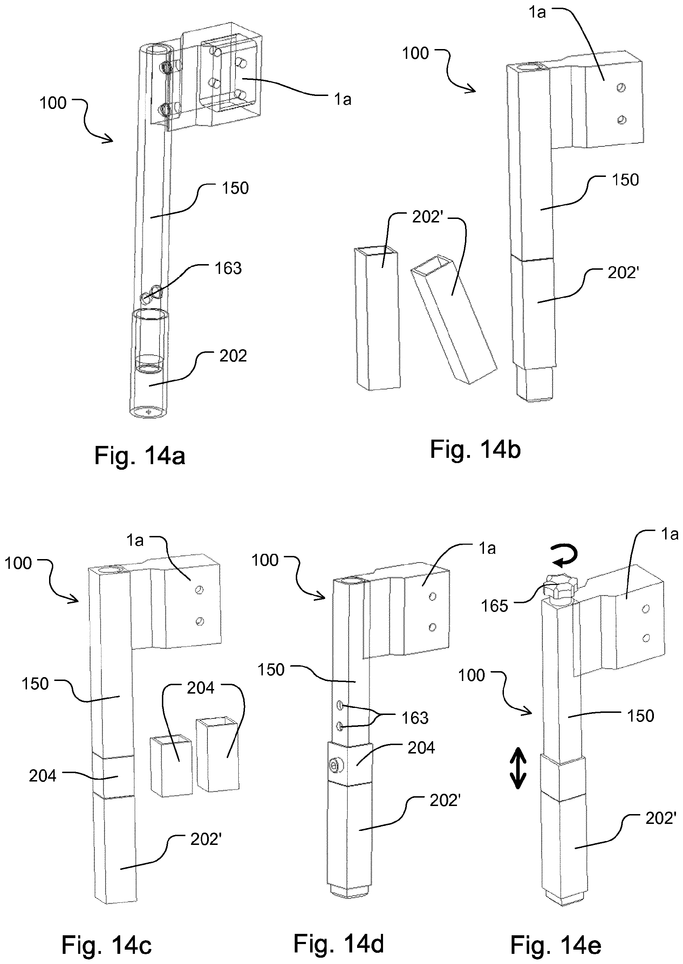

**Fig. 14a** zeigt einen Steckabschnitt 150 für eine erfindungsgemäße Positioniervorrichtung 100 einer dritten Ausführungsform. Die Längsachsen der zylindrischen Hülse 202 und des Steckabschnitts 150 fallen in dieser Ausführungsform zusammen. Die Hülse 202 wird auf den Steckabschnitt 150 aufgeschoben, beispielsweise mittels einer Passung zwischen dem Außendurchmesser der Hülse 202 und dem Innendurchmesser des Steckabschnitts 150.

Um ein gegenseitiges Verdrehen des Steckabschnitts 150 und der Hülse 202 zu verhindern, kann ein Pin, ein Passstift, eine Kerbe in einem Absatz oder ähnlich vorzugsweise am Außendurchmesser des Steckabschnitts 150 und am Innendurchmesser der Hülse 202 angeordnet sein.

Zum Begrenzen der axialen Verschiebbarkeit der Hülse 202 auf dem Steckabschnitt 150 kann ein Stift oder Ähnliches in optionale Querbohrungen 163 des Steckabschnitts 150 eingebracht werden. Ein derartiger Stift kann als axialer Anschlag der Hülse 202 verstanden werden. Mittels eines solchen axialen Anschlags kann die Höhe oder Distanz eines Instruments 23 in Bezug auf die Platte 300 eingestellt werden (siehe Fig. 7).

**Fig. 14b** zeigt einen eckigen Steckabschnitt 150 für eine erfindungsgemäße Positioniervorrichtung 100 einer weiteren Ausführungsform mit eckigen Hülsen 202'. Die Hülsen 202' können optional unterschiedliche Längen aufweisen, um die Höhe (siehe die Beschreibung zur Fig. 14a) einzustellen.

**Fig. 14c** zeigt einen eckigen Steckabschnitt 150 analog zur Ausführungsform der Fig. 14b für eine erfindungsgemäße Positioniervorrichtung 100 einer weiteren Ausführungsform mit Zwischenhülsen 204. Mittels einer oder mehrerer Zwischenhülsen 204 kann die Höhe (siehe die Beschreibung zur Fig. 14a) eingestellt werden.

**Fig. 14d** zeigt einen eckigen Steckabschnitt 150 für eine erfindungsgemäße Positioniervorrichtung 100 einer weiteren Ausführungsform mit einer arretierbaren Zwischenhülse 204. Die Zwischenhülse 204 kann mittels eines Stifts durch den Absatz außen am Zwischenstück 204 und den Querbohrungen 163 axial fixiert werden.

**Fig. 14e** zeigt einen eckigen Steckabschnitt 150 für eine erfindungsgemäße Positioniervorrichtung 100 einer weiteren Ausführungsform mit einer Arretiervorrichtung 165. Die Arretiervorrichtung 165 kann mittels einer Drehung fixiert und gelöst werden, so dass, beispielsweise mittels eines innen (in dem Steckabschnitt 105 und in der Hülse 202') angeordneten Konus die Hülse 202' axial fixiert werden kann.

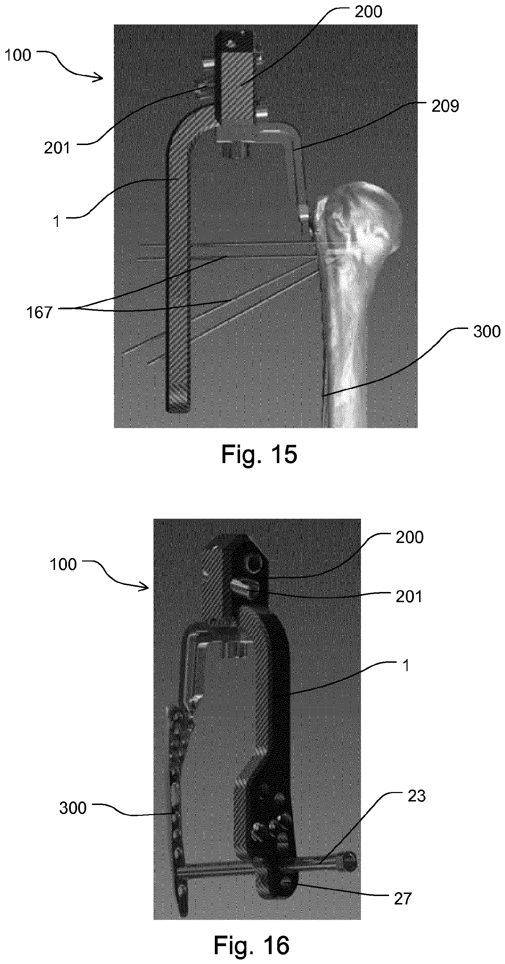

**Fig. 15** zeigt die erfindungsgemäße Positioniervorrichtung 100 zum Führen von K-Drähten (Kirschner-Drähten) in einer Seitenansicht. Mittels K-Drähten können Platten 300 nach Knochenfrakturen am Knochen fixiert werden.

Die am Knochen fixierte Platte 300 ist mit einem Plattenhalter 209 verbunden. Die Verbindung kann eine Schraubverbindung, eine formschlüssige und/oder reibschlüssige Verbindung sein. Die Verbindung kann eine Sollbruchstelle aufweisen, so dass nach einem Positionieren und Fixieren der Platte 300 am Knochen der Plattenhalter 209 abgeknickt und damit abgebrochen und von der Platte 300 vorteilhaft einfach und schnell gelöst werden kann.

Der Plattenhalter 209 ist mit einer Steckhülsenanordnung 200 verbunden, beispielsweise mittels einer Schraubverbindung. Die Steckhülsenanordnung 200 weist mehrere Hülsen 201 auf, in die der Führungsbogen 1, je nach gewünschter Positionierung, eingesteckt werden kann. Mit dieser Positionierung ist die Lage des Führungsbogens 1 relativ zur Platte 300 und zum Knochen festgelegt. Anschließend können die K-Drähte zur Knochenfixierung eingebracht werden.

**Fig. 16** zeigt die erfindungsgemäße Positioniervorrichtung 100 in einer weiteren Ausführungsform. Anders als in der Ausführungsform aus Fig. 15 werden in Fig. 16 jedoch keine K-Drähte zur Knochenfixierung in den Führungsbogen 1 eingebracht, sondern Instrumente 23. Mittels derartiger Instrumente 23 können Schrauben zum Fixieren der Platte 300 am Knochen eingeschraubt werden. Die Instrumente 23 werden wahlweise und je nach gewünschter Fixierung und Verschraubung der Platte 300 am Knochen in eine von vorgebohrten Positionierhilfen 27 in dem Führungsbogen 1 eingeführt.

### Bezugszeichenliste

- 1: Führungsbogen
- 1a: erster Abschnitt
- 1b: zweiter Abschnitt
- 3: Verstellvorrichtung
- 5: Zielvorrichtung
- 21: Verriegelungsvorrichtung
- 23: Instrument
- 27: Positionierhilfe
- 67: Drehspanner

- 100, 100', 100", 100"': Positioniervorrichtung
- 101: Schnittstelle
- 103: Bolzen oder Stifte
- 109: konzentrische Ringe
- 111: Längsschlitz
- 113: Längsschlitz
- 129: Drehrichtung
- 131: Noppen
- 133: Radius
- 135: Radius
- 150: Steckabschnitt
- 151, 153, 155: Führungsstift
- 156: Durchgangsöffnung
- 157: Führungsstift
- 159: Stab
- 161: Handelement
- 163: Querbohrung
- 165: Arretiervorrichtung

- 200: Steckhülsenanordnung
- 201, 203, 205, 207, 208: Hülsen
- 201', 203', 205', 207', 208': Längsnut
- 201a, 203a, 205a, 207a: Passabschnitte
- 204: Zwischenhülse
- 208b: Abschnitt in Umfangsrichtung
- 209: Plattenhalter
- 211: erste Verbindung
- 213: zweite Verbindung
- 300: Platte
- 301, 303, 305, 307, 308: Durchgangsöffnungen

## Patentansprüche

1. Positioniervorrichtung (100, 100', 100", 100"') zum Positionieren und/oder Fixieren einer Platte (300) an einem Knochen, umfassend
- einen Führungsbogen (1), wobei der Führungsbogen (1) in wenigstens einem Abschnitt als ein Steckabschnitt (150) zum Einstecken in eine Hülse (201, 203, 205, 207, 208) einer Steckhülsenanordnung (200) ausgestaltet ist oder einen solchen Steckabschnitt (150) aufweist;
wobei der Führungsbogen (1) eine Verstellvorrichtung (3) aufweist, wobei die Verstellvorrichtung (3) wenigstens eine Zielvorrichtung (5) aufweist, und wobei die Zielvorrichtung (5) ausgebildet ist, um eine Verriegelungsvorrichtung (21) oder ein Instrument (23) zum Einwirken auf die Verriegelungsvorrichtung (21) zumindest abschnittsweise aufzunehmen;
**dadurch gekennzeichnet, dass** die Positioniervorrichtung (100, 100', 100", 100"') weiterhin Steckhülsenanordnung (200) mit einer Vielzahl von Hülsen (201, 203, 205, 207, 208) zum Einstecken des Steckabschnitts (150) umfasst.

2. Positioniervorrichtung (100, 100', 100", 100"') nach Anspruch 1, wobei die Hülsen (201, 203, 205, 207, 208) jeweils eine Längsachse aufweisen, und wobei nicht alle Längsachsen der Hülsen (201, 203, 205, 207, 208) parallel zueinander stehen.

3. Positioniervorrichtung (100, 100', 100", 100"') nach Anspruch 1 oder 2, wobei die Steckhülsenanordnung (200) einen mit ihr verbundenen Plattenhalter (209) aufweist, wobei sich der Plattenhalter (209) vorzugsweise von der Steckhülsenanordnung (200) erstreckt.

4. Positioniervorrichtung (100, 100', 100", 100"') nach Anspruch 3, wobei der Plattenhalter (209) eine erste Verbindung (211) zu seiner lösbaren Verbindung mit einem weiteren Abschnitt der Steckhülsenanordnung (200) aufweist.

5. Positioniervorrichtung (100, 100', 100", 100"') nach einem der Ansprüche 3 oder 4, wobei der Plattenhalter (209) eine zweite Verbindung (213) zu seiner Verbindung mit einer Platte (300) aufweist.

6. Positioniervorrichtung (100, 100', 100", 100"') nach einem der vorangegangenen Ansprüche, wobei der Steckabschnitt (150) wenigstens einen sich radial hiervon erstreckenden Führungsstift (151) aufweist zum Führen des Steckabschnitts (150) in einer in der Vielzahl der Hülsen (201, 203, 205, 207, 208) jeweils ausgestalteten Längsnut (201', 203', 205', 207', 208').

7. Positioniervorrichtung (100, 100', 100", 100"') nach einem der vorangegangenen Ansprüche, wobei die Vielzahl von Hülsen (201, 203, 205, 207, 208) wenigstens je eine Längsnut (201', 203', 205', 207', 208') aufweist.

8. Positioniervorrichtung (100, 100', 100", 100"') nach Anspruch 7, wobei sich die Längsnut (201', 203', 205', 208') nicht über die gesamte Länge der Hülse (201, 203, 205, 208') erstreckt und/oder wobei eine Breite der Längsnut (201', 203', 207') nicht über die gesamte Länge der Hülse (201, 203, 207) konstant ist.

9. Positioniervorrichtung (100, 100', 100", 100"') nach Anspruch 7 oder 8, wobei die Längsnut (203', 205', 208') wenigstens abschnittsweise in Umfangsrichtung der Hülse (203, 205, 208) verläuft.

10. Positioniervorrichtung (100, 100', 100", 100"') nach Anspruch 7, 8 oder 9, wobei die Längsnut (203', 205', 208') wenigstens einen Paßabschnitt hat, welcher mit dem Führungsstift (151) eine vorbestimmte Passung ergibt.

11. Positioniervorrichtung (100, 100', 100") nach einem der vorangegangenen Ansprüche, wobei die Vielzahl von Hülsen (201, 203, 205, 207, 208) mittels Markierung auf der Steckhülsenanordnung (200) gekennzeichnet ist.

12. Positioniervorrichtung (100, 100', 100", 100"') nach einem der vorangegangenen Ansprüche, wobei die Vielzahl von Hülsen (201, 203, 205, 207, 208) mit anderen Abschnitten der Steckhülsenanordnung (200) verschraubt, mittels Passung verbunden, mittels Kleber und/oder mittels Passstift verbunden sind.

13. Positioniervorrichtung (100, 100', 100", 100"') nach einem der vorangegangenen Ansprüche, wobei der Steckabschnitt (150) in seiner Mantelfläche eine Durchgangsöffnung (156) zum Durchführen eines Führungsstifts (157) aufweist, wobei der Steckabschnitt (150) in einem Inneren hiervon wenigstens eine Längsöffnung aufweist, wobei im Inneren oder in der Längsöffnung ein Stab (159) drehbar angeordnet ist, welcher auf seiner Mantelfläche den Führungsstift (157) trägt oder von welcher der Führungsstift (157) wegsteht, wobei der Führungsstift (157) durch die Durchgangsöffnung (156) geführt ist.

14. Positioniervorrichtung (100, 100', 100", 100"') nach Anspruch 13, wobei der Stab (159) ein Handelement (161) zum Drehen des Stabs (159) von Hand innerhalb der Längsöffnung des Steckabschnitts (150) aufweist.

15. Positioniervorrichtung (100, 100', 100", 100"') nach einem der vorangegangenen Ansprüche, verbunden mit der Steckhülsenanordnung (200), wobei die Steckhülsenanordnung (200) verbunden ist mit einer Platte (300) für die Osteosynthese, wobei die Platte (300) eine Vielzahl von Durchgangsöffnungen (301, 303, 305, 307, 308) zur Aufnahme von einer oder mehreren Verriegelungsvorrichtungen (21) aufweist, wobei die Tiefe der Durchgangsöffnungen (301, 303, 305, 307, 308) jeweils einer Dicke der Platte (300) entspricht, wobei eine Vielzahl der Hülsen (201, 203, 205, 207, 208) jeweils einer der Durchgangsöffnungen (301, 303, 305, 307, 308) zugeordnet ist, wobei die Verstellvorrichtung (3) bei in eine der Hülsen (201, 203, 205, 207, 208) eingeführtem Steckabschnitt (150) derart ausgestaltet ist, dass die in der Verstellvorrichtung (3) aufgenommene Zielvorrichtung (5) ausgerichtet und bewegbar ist, dass eine in der Zielvorrichtung (5) aufgenommene Verriegelungsvorrichtung (21) oder ein in der Zielvorrichtung (5) aufgenommenes Instrument (23), insbesondere unter Umschreibung oder innerhalb eines Kegelmantels bewegbar ist, wobei die Kegelspitze innerhalb der Tiefe der Durchgangsöffnung jener Hülse, in welche der Steckabschnitt (150) eingeführt ist, zum Liegen kommt.

## Claims

1. A positioning device (100, 100', 100", 100"') for positioning and/or fixing a plate (300) to a bone, comprising:
- a guiding bow (1), wherein the guiding bow (1) is designed at least in one section as a push-in section (150) to be inserted into a sleeve (201, 203, 205, 207, 208) of a push-in sleeves arrangement (200) or comprises such a push-in section (150);
wherein the guiding bow (1) comprises an adjusting device (3), the adjusting device (3) comprising at least one targeting device (5), and wherein the targeting device (5) is designed to at least partially receive an interlocking device (21) or an instrument (23) for acting on the interlocking device (21);
**characterized in that** the positioning device (100, 100', 100", 100"') further includes a push-in sleeves arrangement (200) comprising a plurality of sleeves (201, 203, 205, 207, 208) for insertion of the push-in section (150).

2. The positioning device (100, 100', 100", 100"') according to claim 1, wherein the sleeves (201, 203, 205, 207, 208) each present a longitudinal axis, and wherein not all longitudinal axes of the sleeves (201, 203, 205, 207, 208) are parallel to each other.

3. The positioning device (100, 100', 100", 100"') according to claim 1 or 2, wherein the push-in sleeves arrangement (200) comprises a plate holder (209) connected thereto, the plate holder (209) preferably extending from the push-in sleeves arrangement (200).

4. The positioning device (100, 100', 100", 100"') according to claim 3, wherein the plate holder (209) comprises a first connection (211) to its detachable connection with a further section of the push-in sleeves arrangement (200).

5. The positioning device (100, 100', 100", 100"') according to anyone of claim 3 or 4, wherein the plate holder (209) comprises a second connection (213) to its connection with a plate (300).

6. The positioning device (100, 100', 100", 100"') according to anyone of the preceding claims, wherein the push-in section (150) comprises at least one guide pin (151) extending radially therefrom for guiding the push-in section (150) in a longitudinal groove (201', 203', 205', 207', 208') designed in each case of the plurality of sleeves (201, 203, 205, 207, 208).

7. The positioning device (100, 100', 100", 100"') according to anyone of the preceding claims, wherein each sleeve of the plurality of sleeves (201, 203, 205, 207, 208) comprises at least one longitudinal groove (201', 203', 205', 208').

8. The positioning device (100, 100', 100", 100"') according to claim 7, wherein the longitudinal groove (201', 203', 205', 208') does not extend over the entire length of the sleeve (201, 203, 205, 208') and/or wherein a width of the longitudinal groove (201', 203', 207') is not constant over the entire length of the sleeve (201, 203, 207).

9. The positioning device (100, 100', 100", 100"') according to claim 7 or 8, wherein the longitudinal groove (203', 205', 208') runs at least partially along the circumferential direction of the sleeve (202, 205, 208).

10. The positioning apparatus (100, 100', 100", 100"') according to claim 7, 8 or 9, wherein the longitudinal groove (203', 205', 208') comprises at least one fitting portion causing a predetermined fit with the guide pin (151).

11. The positioning device (100, 100', 100", 100"') according to anyone of the preceding claims, wherein the plurality of sleeves (201, 203, 205, 207, 208) is identified by means of a marking on the push-in sleeves arrangement (200).

12. The positioning device (100, 100', 100", 100"') according to anyone of the preceding claims, wherein the plurality of sleeves (201, 203, 205, 207, 208) is screwed to other sections of the push-in sleeves arrangement (200), is connected by means of a fit, is connected by means of adhesive and/or by means of a fitting pin.

13. The positioning device (100, 100', 100", 100"') according to anyone of the preceding claims, wherein the push-in section (150) comprises a through-opening (156) for the passage of a guide pin (157) in its peripheral surface, the push-in section (150) comprising at least one longitudinal opening in an interior thereof, a rod (159) being rotatably arranged in the interior or in the longitudinal opening, wherein the rod carries the guide pin (157) on its peripheral surface or from which the guide pin (157) sets away, the guide pin (157) being guided through the through-opening (156).

14. The positioning apparatus (100, 100', 100", 100"') according to claim 13, wherein the rod (159) comprises a hand element (161) for manually rotating the rod (159) within the longitudinal opening of the push-in section (150).

15. The positioning device (100, 100', 100", 100"') according to anyone of the preceding claims, connected with the push-in sleeves arrangement (200), wherein the push-in sleeves arrangement (200) is connected with a plate (300) for the osteosynthesis, the plate (300) comprising a plurality of through openings (301, 303, 305, 307, 308) for accommodating one or more interlocking devices (21), the depth of the through openings (301, 303, 305, 307, 308) each time corresponding to a thickness of the plate (300), each sleeve of the plurality of the sleeves (201, 203, 205, 207, 208) being assigned to one of the through openings (301, 303, 305, 307, 308), the adjusting device (3) being designed by a push-in section (150) inserted in one of the sleeves sleeves (201, 203, 205, 207, 208) so that the targeting device (5) accommodated in the adjusting device (3) is aligned and movable, so that one interlocking device (21) accommodated in the targeting device (5) or one instrument (23) accommodated in the targeting device (5) is movable, in particular by circling around or within a conical envelope, the conical tip coming to rest within the depth of the through opening of that sleeve into which the push-in section (150) is inserted.

## Revendications

1. Un dispositif de positionnement (100, 100', 100", 100"') permettant de positionner et/ou de fixer une plaque (300) sur un os, comprenant:
- un arceau de guidage (1), l'arceau de guidage (1) étant conçu dans au moins une section comme une section enfichable (150) à insérer dans un manchon (201, 203, 205, 207, 208) d'un agencement de manchons enfichables (200) ou comprenant une telle section enfichable (150);
où l'arceau de guidage (1) comprend un dispositif d'ajustement (3), le dispositif d'ajustement (3) comprenant au moins un dispositif de visée (5), et le dispositif de visée (5) étant conçu pour recevoir un dispositif de verrouillage (21) ou un instrument (23) permettant d'agir au moins partiellement sur le dispositif de verrouillage (21);
**caractérisé en ce que** le dispositif de positionnement (100, 100', 100", 100"') inclut en outre un agencement de manchons enfichables (200) ayant une pluralité de manchons (201, 203, 205, 207, 208) pour l'insertion dans la section enfichable (150).

2. Le dispositif de positionnement (100, 100', 100", 100"') selon la première revendication, où les manchons (201, 203, 205, 207, 208) comportent chacun un axe longitudinal, et où tous les axes longitudinaux des manchons (201, 203, 205, 207, 208) ne sont pas parallèles les uns aux autres.

3. Le dispositif de positionnement (100, 100', 100", 100"') selon la revendication 1 ou 2, où l'agencement de manchons enfichables (200) comprend un support de plaque (209) relié à celui-ci, le support de plaque (209) s'étendant de préférence depuis l'agencement de manchons enfichables (200).

4. Le dispositif de positionnement (100, 100', 100", 100"') selon la revendication 3, où le support de plaque (209) comprend un premier raccord (211) pour son raccordement amovible avec une autre section de l'agencement de manchons enfichables (200).

5. Le dispositif de positionnement (100, 100', 100", 100"') selon l'une quelconque des revendications 3 ou 4, où le support de plaque (209) comprend un second raccord (213) pour son raccordement avec une plaque (300).

6. Le dispositif de positionnement (100, 100', 100", 100"') selon l'une quelconque des revendications précédentes, où la section enfichable (150) comprend au moins une broche de guidage (151) s'étendant radialement depuis celle-ci pour guider la section enfichable (150) dans une des rainures longitudinales (201', 203', 205', 207', 208') conçue dans la pluralité de manchons (201, 203, 205, 207, 208).

7. Le dispositif de positionnement (100, 100', 100", 100"') selon l'une quelconque des revendications précédentes, où chaque manchon de la pluralité de manchons (201, 203, 205, 207, 208) comprend au moins une rainure longitudinale (201', 203', 205', 208') .

8. Le dispositif de positionnement (100, 100', 100", 100"') selon la revendication 7, où la rainure longitudinale (201', 203', 205', 208') ne s'étend pas sur toute la longueur du manchon (201, 203, 205, 208') et/ou où une largeur de la rainure longitudinale (201', 203', 207') n'est pas constante sur toute la longueur du manchon (201, 203, 207).

9. Le dispositif de positionnement (100, 100', 100", 100"') selon la revendication 7 ou 8, où la rainure longitudinale (203', 205', 208') s'étend au moins partiellement dans la direction circonférentielle du manchon (202, 205, 208).

10. Le dispositif de positionnement (100, 100', 100", 100"') selon la revendication 7, 8 ou 9, où la rainure longitudinale (203', 205', 208') comprend au moins une section d'ajustement, laquelle engendre un ajustement prédéterminé avec la broche de guidage (151).

11. Le dispositif de positionnement (100, 100', 100", 100"') selon l'une quelconque des revendications précédentes, où la pluralité de manchons (201, 203, 205, 207, 208) est identifiée au moyen d'un marquage sur l'agencement de manchons enfichables (200).

12. Le dispositif de positionnement (100, 100', 100", 100"') selon l'une quelconque des revendications précédentes, où la pluralité de manchons (201, 203, 205, 207, 208) est vissée, reliée par ajustement, reliée au moyen d'adhésif et/ou d'une broche d'ajustement à d'autres sections de l'agencement de manchons enfichables (200).

13. Le dispositif de positionnement (100, 100', 100", 100"') selon l'une quelconque des revendications précédentes, où la section enfichable (150) comprend dans sa surface périphérique une ouverture de passage (156) pour le passage d'une broche de guidage (157), la section enfichable (150) comprenant au moins une ouverture longitudinale dans son intérieur, une tige (159) étant agencée de manière pivotable à l'intérieur ou dans l'ouverture longitudinale, la tige portant la broche de guidage (157) sur sa surface périphérique ou la broche de guidage (157) s'éloignant de la tige, la broche de guidage (157) étant guidée à travers l'ouverture de passage (156).

14. Le dispositif de positionnement (100, 100', 100", 100"') selon la revendication 13, où la tige (159) comprend un élément manuel (161) pour faire tourner manuellement la tige (159) à l'intérieur de l'ouverture longitudinale de la section enfichable (150).

15. Le dispositif de positionnement (100, 100', 100", 100"') selon l'une quelconque des revendications précédentes relié à l'agencement de manchons enfichables (200), où l'agencement de manchons enfichables (200) est relié à une plaque (300) pour l'ostéosynthèse, la plaque (300) comprenant une pluralité d'ouvertures de passage (301, 303, 305, 307, 308) pour la réception d'un ou plusieurs appareils de verrouillage (21), la profondeur des ouvertures de passage (301, 303, 305, 307, 308) correspondant respectivement à une épaisseur de la plaque (300), une pluralité de manchons (201, 203, 205, 207, 208) étant respectivement attribuée à l'une des ouvertures de passage (301, 303, 305, 307, 308), le dispositif d'ajustement (3) étant conçu par une section enfichable (150) insérée dans l'un des manchons (201, 203, 205, 207, 208) de telle sorte que le dispositif de visée (5) logé dans le dispositif d'ajustement (3) est alignable et mobile, de telle sorte qu'un dispositif de verrouillage (21) logé dans le dispositif de visée (5) ou un instrument (23) logé dans le dispositif de visée (5) puisse être mobile, notamment en cercle ou à l'intérieur d'une enveloppe conique, la pointe du cône venant s'arrêter dans la profondeur de l'ouverture de passage du manchon dans lequel la section enfichable (150) est insérée.
